# EUROPÄISCHE PATENTSCHRIFT

(11) **EP 1 926 697 B1**
(45) Veröffentlichungstag und Bekanntmachung des Hinweises auf die Patenterteilung: **04.05.2016**
(21) Anmeldenummer: 06793143.6
(22) Anmeldetag: 01.09.2006
(51) Int. Cl.: C07C 31/30, C07C 29/70

(54) **MAGNESIUMALKOXIDGRANULAT, VERFAHREN ZU DESSEN HERSTELLUNG UND VERWENDUNG DES MAGNESIUMALKOXIDGRANULATES**
MAGNESIUM ALKOXIDE GRANULATE, METHOD FOR THE PRODUCTION THEREOF, AND USE THEREOF
GRANULAT D'ALCOXYDE DE MAGNESIUM, PROCEDE PERMETTANT DE LE PRODUIRE ET UTILISATION DUDIT GRANULAT D'ALCOXYDE DE MAGNESIUM

(30) Priorität: 01.09.2005 DE 102005041774
(43) Veröffentlichungstag der Anmeldung: 04.06.2008
(73) Patentinhaber: Chemetall GmbH, 60487 Frankfurt am Main (DE)
(72) Erfinder: DIETZ, Rainer, 63329 Egelsbach (DE); EMMEL, Ute, 65929 Frankfurt Am Main (DE); WIETELMANN, Ulrich, Dr., 61381 Friedrichsdorf (DE); KRÄMER, Gerd, 61118 Bad Vilbel (DE)
(74) Vertreter: Uppena, Franz
(86) Internationale Anmeldenummer: PCT/EP2006/065918
(87) Internationale Veröffentlichungsnummer: WO 2007/026017

(56) Entgegenhaltungen:
- EP-A1- 0 144 021
- EP-A2- 0 997 451
- EP-A2- 1 306 360
- LIN J-M ET AL: "Synthesis of Benzyl/Allyl Alkyl Ethers from Corresponding Magnesium Alkoxides" TETRAHEDRON LETTERS, ELSEVIER, AMSTERDAM, NL, Bd. 37, Nr. 29, 15. Juli 1996 (1996-07-15), Seiten 5159-5160, XP004029615 ISSN: 0040-4039 in der Anmeldung erwähnt

## Beschreibung

Gegenstand der vorliegenden Erfindung sind Magnesiumalkoxidgranulate, ein Verfahren zu deren Herstellung sowie deren Verwendung.

Polyolefine werden typischerweise aus Olefin-Monomeren mit Hilfe eines organometallischen Katalysatorsystems vom Ziegler-Natta-Typ hergestellt. Ziegler-Natta-Katalysatoren bestehen allgemein aus Komplexen, die aus dem Halogenid eines Übergangsmetalles, typischerweise Titan, Chrom oder Vanadium und einem Aluminiumalkyl gebildet werden. Üblicherweise werden diese Komplexe durch Aufbringen auf eine unlösliche Magnesiumverbindung, beispielsweise ein Magnesiumalkoxid wie Magnesiumethoxid, heterogenisiert.

An der Oberfläche des Magnesiumalkoxides läuft ein Austauschprozess mit dem Übergangsmetallchlorid ab, beispielsweise:

Mg(OEt)₂ + Cp₂TiCl₂ → Mg(OEt)ₓCl₂₋ₓ + Cp₂TiClₓTi₂₋ₓ

mit x = 0 oder 1 und Cp = Cyclopentadienyl (EP-A-1 031 580).

Das als Trägermaterial und Halogenakzeptor fungierende Magnesiumalkoxid muss im Sinne einer industriellen Verwendbarkeit einige physikalische Rahmenbedingungen erfüllen:
a) es sollte eine möglichst hohe Oberfläche aufweisen, damit eine entsprechend hohe Katalysatoraktivität erreicht werden kann;
b) es sollte staubfrei sein und gut fließfähige Feststoffpartikel enthalten;
c) es sollte aus möglichst ähnlich großen Partikeln bestehen, also eine enge Partikelgrößenverteilung aufweisen, damit eine gleichmäßige Polymerisation erreicht wird, und
d) es sollte möglichst keine den Katalyseprozess störenden Verunreinigungen wie beispielsweise lod enthalten.

Magnesiumalkoxide der allgemeinen Formel Mg(OR)₂ können durch Umsetzung von Magnesiummetall mit dem jeweiligen Alkohol der allgemeinen Formel ROH unter Rückflussbedingungen hergestellt werden. Um die Umsetzung zu beschleunigen, bedarf es eines Katalysators, typischerweise lod, der in Mengen von etwa 1 mol% zugegeben wird. Trotzdem dauert die Reaktion noch sehr lange. So werden beispielsweise für die Herstellung von Magnesiumethoxid beim Einsatz von Magnesiumpulver etwa 11 Stunden (JP-A-03074341) bzw. bis zu 24 Stunden (Tetrahedron Lett. 37, (1996) 5159-5160) benötigt.

Bei diesen langen Reaktionszeiten zerfallen die gebildeten Magnesiumethoxidpartikel in der Regel zu einem feinen Pulver. Es lassen sich allenfalls sphärische Partikel mit sehr kleinen Durchmessern, beispielsweise 21,9 µm erzeugen (JP-A-03074341). Um die Reaktionsgeschwindigkeit zu erhöhen und ein Produkt mit größeren Partikeln, beispielsweise größer 500 µm, herstellen zu können, kann die Reaktion in purem Alkohol in einem Autoklaven unter erhöhtem Druck und damit möglichst erhöhter Temperatur hergestellt werden (EP-A-997 451). Diese Synthesevariante hat den Nachteil, dass spezielle, aufwändige Apparate benötigt werden. Außerdem sind die entstehenden Magnesiumethoxidpartikel mit überschüssigem Alkoholat gesättigt.

Da bei der Herstellung und späteren Verwendung der Ziegler-Natta-Katalysatoren keine protischen Stoffe wie beispielsweise Ethanol anwesend sein dürfen, muss das grobkörnige Produkt sorgfältig gewaschen und getrocknet werden.

Es ist weiterhin bekannt, die Umsetzung vom Magnesiummetall mit Ethanol in Gegenwart von Kohlenwasserstofflösemitteln durchzuführen. US-A-4,959,336 offenbart Umsetzungen von Magnesiumpulver in Gegenwart von Toluol, wobei als Katalysator eine Mischung aus Brom und Eisenchlorid eingesetzt wird und der Alkohol Ethanol bereits während der Anfangsphase der Synthese im Überschuss, beispielsweise zwischen 86 % und 270 %, eingesetzt wird. Weitere erhebliche Alkoholmengen werden nach einigen Stunden Reaktionszeit zugegeben. Die Umsetzung wird durch Refluxieren in Gegenwart von Titanisopropoxid vervollständigt. Nachteile dieses Verfahrens sind die Vielstufigkeit und die langen Herstellzeiten, beispielsweise einige Tage bei Normaldruck oder etwa ein Tag bzw. mindestens 6 Stunden bei der Drucksynthese im Autoklaven. Außerdem ist das Endprodukt mit unerwünschten Komponenten aus der Katalysatormischung verunreinigt.

Aufgabe der vorliegenden Erfindung ist es, die Nachteile des Standes der Technik zu überwinden.

Erfindungsgemäß wird die Aufgabe durch die Merkmale des Hauptanspruchs gelöst. Vorzugsweise Ausgestaltungen finden sich in den Unteransprüchen.

Insbesondere ist es Aufgabe der vorliegenden Erfindung, ein Verfahren zur Herstellung eines Magnesiumalkoxidgranulats mit einstellbarer Partikelgröße bereitzustellen.
1. Dabei werden erfindungsgemäß die Aufgaben dadurch gelöst, dass Magnesium mit Magnesiumpartikelgrößen von 0,3 bis 5 mm mit 0,001 bis über 20 mol% mit einer Verbindung oder mehreren Verbindungen der allgemeinen Formel AIR₃₋ₙHalₙ, wobei
   - der Rest R als Alkyl- oder Aryl- Rest
   - der Rest Hal als Halogen-Rest und
   - n als Zahl, wobei 0 ≤ n ≤ 2 ist, aktiviert und einem Überschuss an Alkohol zwischen 0,1 und 60 % in nicht-koordinierenden Lösemitteln umgesetzt wird mit folgenden Verfahrensschritten:
   - Vorlage des Lösemittels und des Maqnesiummetalls in einen mit Schutzgas gefüllten Rührreaktor;
   - Zugabe einer oder mehrerer Verbindungen der allgemeinen Formel AlR₃₋ₙHalₙ;
   - Zugabe des Alkohols. Das Magnesiummetall wird in aromatischen Kohlenwasserstofflösemitteln mit primären Alkoholen bei Temperaturen zwischen etwa Raumtemperatur und dem Siedepunkt des jeweiligen Alkohols umgesetzt, wobei der Alkohol mit geringem Überschuss, beispielsweise zwischen 0,1 und 60 % eingesetzt wird. Das eingesetzte Magnesiummetall kann mit bekannten Methoden wie beispielsweise lod oder Brom/Eisenchlorid aktiviert werden. Bevorzugt wird zur Aktivierung das Magnesiummetall in Kohlenwasserstoffsuspension mit geringen Mengen, beispielsweise 0,001 bis 2 mol%, bevorzugt 0,1 bis 1,5 mol% eines Aluminiumalkyls vorbehandelt. Es können prinzipiell aber auch höhere Mengen an Aluminiumalkyl, beispielsweise 10 oder sogar über 20 mol% eingesetzt werden. Die erhöhte Zugabe von Aluminiumalkyl hat den Vorteil, dass die Viskosität der Alkoxidlösungen erniedrigt wird. Dies macht diese Lösungen leichter förderbar.

Es wurde überraschend gefunden, dass das Magnesiumalkoxidgranulat nach dem erfindungsgemäßem Verfahren deutlich schneller gebildet wird, als dies nach dem Stand der Technik, beispielsweise in purem Alkohol oder in einer Kohlenwasserstoff/Alkoholmischung mit deutlich überschüssigem Alkohol, der Fall ist. Außerdem wurde überraschend gefunden, dass die nach dem erfindungsgemäßen Verfahren hergestellten gebildeten Partikel eine verbesserte mechanische Festigkeit aufweisen, das heißt, dass sie beim Syntheseprozess nicht zerfallen. Deshalb ist der Staubanteil, das heißt der Anteil an Endprodukt, dessen Korngröße < 0,2 mm ist, sehr gering und es wird eine enge Partikelgrößenverteilung beobachtet.

Der Partikeldurchmesser lässt sich durch Auswahl entsprechender Magnesiummetallqualitäten steuern, wie in Tabelle 1 aufgeführt:

**Tabelle 1: Magnesiumqualitäten**

| Mg-Qualität | Aussehen | Partikelabmessungen Mg - Metall |
|---|---|---|
| A | Feinspäne | 1 mm breit, 3 - 6 mm lang |
| B | Granalien | Ø 0,5 - 1 mm, |
| C | Grobspäne | 3 mm breit, 3 - 10 mm lang |

Durch Umsetzung mit primären Alkoholen, beispielsweise Ethanol, unter erfindungsgemäßen Bedingungen entstehen oberflächenreiche Granulate mit zum Teil weitgehend sphärischen Abformungen.

**Tabelle 2: Produkteigenschaften**

| Mg-Einsatz | Anteil in Gew.-% | | | Abbildung (Fig.1) | Beispiel Nr. |
|---|---|---|---|---|---|
| | Siebanalyse (mm) | | | | |
| | 0 - 0,5 | 0,5 - 1,6 | > 1,6 | | |
| Qualität A | 45 | 54 | 1 | 1 | 1 |
| Qualität B | 0,5 | 99 | 0,5 | 2 | 2 |
| Qualität C | 1 | 6 | 93 | 3 | 3 |
| Qualität A | 92 | 7 | 1 | 4 | Vogl. 1 |

Das erfindungsgemäße Verfahren zur Herstellung des Magnesiumalkoxidgranulats wird allgemein wie nachfolgend beschrieben durchgeführt, ohne die Erfindung darauf einzuschränken:

In einem inertisierten, das heißt in ein trockenen und mit Schutzgas, beispielsweise Stickstoff oder Argon, gefülltem Rührreaktor werden ein aromatisches Kohlenwasserstofflösemittel und Magnesiummetall vorgelegt, wobei der aromatische Kohlenwasserstoff 6 bis 15 Kohlenstoffatome besitzt. Bevorzugte Lösemittel sind: Benzol, Toluol, Ethylbenzol, Cumol oder Xylole, entweder in reiner Form oder in Mischung von mindestens zwei dieser Lösemittel. Das Magnesium wird in handelsüblicher Form, das heißt in Form von Pulver, Granalien oder Spänen eingesetzt. Die Auswahl der Partikelgröße erfolgt unter Berücksichtigung des gewünschten Teilchendurchmessers des Magnesiumalkoxids. Im Allgemeinen sind Magnesiumpartikelgrößen von 0,3 bis 5 mm bevorzugt; besonders bevorzugt sind Magnesiumpartikelgrößen von 1 bis 1,5 mm Für die Herstellung runder, also sphärischer Partikel wird bevorzugt ein granuliertes Magnesium verwendet, beispielsweise Qualität B aus Tabelle 1. Granulierte, bereits weitgehend sphärische Metallabformungen haben den Vorteil, dass auch das Magnesiumalkoxidgranulat in ähnlicher Form resultiert. Außerdem wird beim Einsatz von solchem Granulat bedeutend weniger, teilweise gar kein staubförmiges Produkt erhalten. Dies ist ein besonderer Vorteil für Anwendungen in der Katalyse, da in diesem Fall ein Granulat mit einer engen Partikelgrößenverteilung gefordert ist.

Die Umsetzung mit einem Alkohol, beispielsweise primären Alkohol, ist aufgrund der das Metall passivierenden Oxidschicht gehemmt. Es muss deshalb eine Aktivierung mit einem Reagenz erfolgen, das die Oxidschicht angreift und so die aktive Metalloberfläche freilegt. Dies kann durch Zugabe bekannter Aktivierungsmittel, also lod, Brom, Eisenchlorid oder dergleichen erfolgen. Bevorzugt ist jedoch die Aktivierung durch Aluminiumverbindungen der allgemeinen Formel AlR₃₋ₙHalₙ, nachfolgend Aktivierungsmittel genannt, wobei hier wie auch nachfolgend zu verstehen ist:
der Rest R als Alkyl- oder Aryl- Rest, bevorzugt als Alkyl-Rest, besonders bevorzugt als Alkyl-Rest mit 1 bis 8 C-Atomen;
der Rest Hal als Halogen-Rest, vorzugsweise als Chlor-, Brom- oder lod-Rest;
n als Zahl, wobei 0 ≤ n ≤ 2 ist.

Besonders bevorzugt ist der Einsatz von Trimethylaluminium (TMA), Triethylaluminium (TEA), Tributylaluminium (TBA), Ethylaluminiumdichlorid (EADC) oder Diethylaluminiumchlorid (DEAC) oder Mischungen von zwei oder mehreren dieser Verbindungen. Diese Verbindungen sind in kommerziellen Mengen als Reinstoffe oder als Lösungen in Kohlenwasserstoffen erhältlich.

Das Aktivierungsmittel wird in bevorzugter Weise zur Suspension des Magnesiummetalls in einem aromatischen Kohlenwasserstoff gegeben, bevor der Alkohol addiert wird. Die Zugabe des Aktivierungsmittels erfolgt bei Temperaturen zwischen 0 und 150°C, bevorzugt zwischen Raumtemperatur und 100°C. Damit die Aktivierung erfolgen kann, ist es notwendig, eine gewisse Kontaktzeit zwischen Magnesiummetall und dem Aktivierungsmittel einzuhalten. Die erforderliche Zeit ist beispielsweise abhängig von der Art der gewählten Magnesiumqualität, das heißt von deren Oberflächenbeschaffenheit. Im Allgemeinen sind Kontaktzeiten zwischen etwa einer Minute und einigen Stunden ausreichend. Um die Aktivierung zu erleichtern, ist es sinnvoll, dabei zu rühren oder in einer anderen Weise zu homogenisieren.

Nach erfolgter Aktivierung kann mit der Alkoholdosierung begonnen werden. Die Dosierung erfolgt je nach Ansatzgröße und sonstigen apparativen Begleitumständen über einen Zeitraum von wenigen Minuten bis einigen Stunden. Typischerweise sind ein bis zwei Stunden sinnvoll. Um eine genügend hohe Reaktionsgeschwindigkeit zu gewährleisten, werden primäre Alkohole verwendet. Vorzugsweise werden Methanol, Ethanol oder Propanol, entweder in reiner Form oder in Mischung, eingesetzt. Die Reaktion ist jedoch grundsätzlich auch mit sekundären oder tertiären Alkoholen möglich, auch in Mischung untereinander oder in Mischung mit primären Alkoholen, insbesondere in Mischung mit Methanol und/oder Ethanol. Der Reaktionsfortschritt kann beispielsweise über die Beobachtung der Gasentwicklung erfolgen. Die Umsetzung erfolgt im Temperaturbereich zwischen Raumtemperatur und etwa 150°C. Da die Reaktion bevorzugt unter Normaldruck gefahren wird, ist die obere Temperaturgrenze durch den Siedepunkt des Lösemittels begrenzt, also beispielsweise 110°C im Falle von Toluol und 136°C im Falle von Ethylbenzol.

Besonders bevorzugt wird die Reaktion am Siedepunkt gefahren. Die Suspension wird dabei bevorzugt vorsichtig, das heißt wenig intensiv gerührt. Ein zu starker mechanischer Energieeintrag kann zu einer Zerstörung der gebildeten Magnesiumalkoxidpartikel führen. Die Menge an aromatischem Kohlenwasserstoff wird so gewählt, dass sich das Magnesiummetall und das daraus gebildete Magnesiumalkoxidgranulat gut rühren lassen. Im Allgemeinen beträgt der Anteil des Magnesiumalkoxids in der fertigen Reaktionsmischung zwischen 5 und 40 Gew-%, bevorzugt zwischen 10 und 30 Gew-%. Der Alkohol wird, bezogen auf die für einen vollständigen Umsatz zum Alkoxid notwendige Menge, mit geringem Überschuss, bevorzugt zwischen 0,1 und 60 % Überschuss, eingesetzt. Besonders bevorzugt beträgt die Alkoholmenge zwischen 101 und 120 % entsprechend ein bis 20 % Überschuss.

Die Kontaktzeit zwischen Magnesiummetall und dem primären Alkohol wird so gewählt, dass ein vollständiger Umsatz des Metalls gewährleistet ist, bevorzugt 0,5 bis 20 Stunden.

Das Gemisch aus Produkt und Kohlenwasserstoff wird in geeigneter Form nach Methoden gemäß dem Stand der Technik aufgearbeitet. Bevorzugt wird das Lösemittel durch Filtration oder Zentrifugation abgetrennt und das feste Magnesiumalkoxidgranulat vakuumgetrocknet. Es ist auch möglich, die flüchtigen Komponenten, das heißt Lösemittel und Restalkohol durch Totaleindampfung zu entfernen. Alle diese Vorgänge werden entweder im Vakuum oder unter Schutzgas ohne unnötigen mechanischen Energieeintrag durchgeführt, um zu vermeiden, dass das Produkt chemisch oder mechanisch zerstört wird.

Abschließend kann das Endprodukt noch gesiebt werden, um den Staubanteil abzutrennen.

Der mittlere Durchmesser d₅₀ der Körner des Magnesiumalkoxidgranulates beträgt 0,3 bis 5 mm, bevorzugt 1 bis 3 mm. Das Magnesiumalkoxidgranulat kann beispielsweise zur Herstellung von Polymerisationskatalysatoren vom Ziegler-Natta-Typ eingesetzt werden.

Gegenstand der Erfindung ist im Einzelnen:
- ein Verfahren zur Herstellung eines Magnesiumalkoxidgranulates durch Umsetzung von Magnesium mit einer Verbindung oder mehreren Verbindungen der allgemeinen Formel AlR₃-ₙHalₙ und einem Alkohol in einem nicht-koordinierenden Lösemittel;
- ein Verfahren zur Herstellung eines Magnesiumalkoxidgranulates durch Umsetzung von Magnesium mit einer Verbindung oder mehreren Verbindungen der allgemeinen Formel AlR₃₋ₙHalₙ und einem Alkohol in einem nicht-koordinierenden Lösemittel, das folgende Verfahrensschritte enthält:
- Vorlage des Lösemittels und des Magnesiummetalls in einen mit Schutzgas gefüllten Rührreaktor;
- Zugabe einer oder mehrerer Verbindungen der allgemeinen Formel AlR₃₋ₙHalₙ;
- Zugabe des Alkohols;
- ein Verfahren zur Herstellung eines Magnesiumalkoxidgranulates durch Umsetzung von Magnesium mit einer Verbindung oder mehreren Verbindungen der allgemeinen Formel AlR₃₋ₙHalₙ und einem Alkohol in einem nicht-koordinierenden Lösemittel, wobei das Magnesium in metallischer Form vorgelegt wird;
- ein Verfahren zur Herstellung eines Magnesiumalkoxidgranulates durch Umsetzung von Magnesium mit einer Verbindung oder mehreren Verbindungen der allgemeinen Formel AlR₃₋ₙHalₙ und einem Alkohol in einem nicht-koordinierenden Lösemittel, wobei das Magnesium in granulierter, sphärischer Form vorgelegt wird;
- ein Verfahren zur Herstellung eines Magnesiumalkoxidgranulates durch Umsetzung von Magnesium mit einer Verbindung oder mehreren Verbindungen der allgemeinen Formel AlR₃₋ₙHalₙ und einem Alkohol in einem nicht-koordinierenden Lösemittel, wobei die Korngröße des Magnesiums zwischen 0,3 und 5 mm, bevorzugt 1 bis 3 mm beträgt;
- ein Verfahren zur Herstellung eines Magnesiumalkoxidgranulates durch Umsetzung von Magnesium mit einer Verbindung oder mehreren Verbindungen der allgemeinen Formel AlR₃₋ₙHalₙ und einem Alkohol in einem nicht-koordinierenden Lösemittel, wobei der Alkohol nach den Verbindungen der allgemeinen Formel AlR₃₋ₙHalₙ zugegeben wird;
- ein Verfahren zur Herstellung eines Magnesiumalkoxidgranulates durch Umsetzung von Magnesium mit einer Verbindung oder mehreren Verbindungen der allgemeinen Formel AlR₃₋ₙHalₙ und einem Alkohol in einem nicht-koordinierenden Lösemittel, wobei der Alkohol ein primärer Alkohol ist;
- ein Verfahren zur Herstellung eines Magnesiumalkoxidgranulates durch Umsetzung von Magnesium mit einer Verbindung oder mehreren Verbindungen der allgemeinen Formel AlR₃₋ₙHalₙ und einem Alkohol in einem nicht-koordinierenden Lösemittel, wobei der Alkohol ausgewählt ist aus Methanol, Ethanol oder Propanol oder Mischungen aus mindestens zwei dieser Alkohole.
- ein Verfahren zur Herstellung eines Magnesiumalkoxidgranulates durch Umsetzung von Magnesium mit einer Verbindung oder mehreren Verbindungen der allgemeinen Formel AlR₃₋ₙHalₙ und einem Alkohol in einem nicht-koordinierenden Lösemittel, wobei der Alkohol Ethanol ist;
- ein Verfahren zur Herstellung eines Magnesiumalkoxidgranulates durch Umsetzung von Magnesium mit einer Verbindung oder mehreren Verbindungen der allgemeinen Formel AlR₃₋ₙHalₙ und einem Alkohol in einem nicht-koordinierenden Lösemittel, wobei der Überschuss des Alkohols zwischen 0,1 und 60 %, bevorzugt zwischen ein und 20 % beträgt;
- ein Verfahren zur Herstellung eines Magnesiumalkoxidgranulates durch Umsetzung von Magnesium mit einer Verbindung oder mehreren Verbindungen der allgemeinen Formel AlR₃₋ₙHalₙ und einem Alkohol in einem nicht-koordinierenden Lösemittel, wobei das Lösemittel ausgewählt ist aus Benzol, Toluol, Ethylbenzol, Cumol, Xylolen oder Mischungen aus mindestens zwei dieser Lösemittel.
- ein Verfahren zur Herstellung eines Magnesiumalkoxidgranulates durch Umsetzung von Magnesium mit einer Verbindung oder mehreren Verbindungen der allgemeinen Formel AlR₃₋ₙHalₙ und einem Alkohol in einem nicht-koordinierenden Lösemittel, wobei das Lösemittel Toluol ist;
- ein Verfahren zur Herstellung eines Magnesiumalkoxidgranulates durch Umsetzung von Magnesium mit einer Verbindung oder mehreren Verbindungen der allgemeinen Formel AlR₃₋ₙHalₙ und einem Alkohol in einem nicht-koordinierenden Lösemittel, wobei die Zugabe des Aktivierungsmittels bei Temperaturen zwischen 0 und 150°C, bevorzugt zwischen Raumtemperatur und 100°C, erfolgt;
- ein Verfahren zur Herstellung eines Magnesiumalkoxidgranulates durch Umsetzung von Magnesium mit einer Verbindung oder mehreren Verbindungen der allgemeinen Formel AlR₃₋ₙHalₙ und einem Alkohol in einem nicht-koordinierenden Lösemittel, wobei der Zeitraum, in dem der Alkohol zudosiert wird, zwischen 3 Minuten und 6 Stunden, bevorzugt zwischen ein und zwei Stunden beträgt;
- ein Verfahren zur Herstellung eines Magnesiumalkoxidgranulates durch Umsetzung von Magnesium mit einer Verbindung oder mehreren Verbindungen der allgemeinen Formel AlR₃₋ₙHalₙ und einem Alkohol in einem nicht-koordinierenden Lösemittel, wobei die Umsetzung des Magnesiums mit dem Alkohol bei Temperaturen zwischen Raumtemperatur und 150°C, bevorzugt zwischen Raumtemperatur und dem Siedepunkt des Lösemittels erfolgt;

Die Erfindung wird nachfolgend durch Beispiele erläutert, ohne sie darauf einzuschränken:

### Beispiel 1: Herstellung eines Magnesiumethoxid-Granulates durch Umsetzung von Magnesium-Feinspänen, Qualität A, mit Ethanol in Toluolsuspension

In einem inertisierten 3-L-Doppelmantelreaktor, versehen mit Propellerrührer, Tropftrichter und Rückflusskühler werden 77,3 g (3180 mmol) Magnesiumfeinspäne (NF2 von Firma Minmet) in 1400 g Toluol vorgelegt. Dann wird unter Rühren auf 50°C erhitzt und 10 ml einer 25 %igen Triethylaluminiumlösung in Toluol zugegeben. Bei der angegebenen Temperatur wird 30 Minuten gerührt und dann mit der Zudosierung von wasserfreiem Ethanol begonnen. Es wurden insgesamt 307 g Ethanol (6660 mmol, 4,7 % Überschuss) über einen Zeitraum von 1,5 Stunden zugegeben. Die Umsetzung springt fast unverzögert an, was an Temperaturerhöhung und Gasentwicklung zu erkennen ist. Durch Gegenkühlung wird die Innentemperatur auf 65°C begrenzt. Bei Zugabeende haben sich 55 L Gas (≙ 72 % d.Th.) entwickelt.

Die Manteltemperatur wird dann auf 90°C erhöht. Nach einer Nachreaktionszeit von 100 Minuten kommt die Gasbildung zum Stillstand. Insgesamt haben sich 75,8 L Gas (≙ 99 % d.Th.) entwickelt.

Die Reaktionsmischung wird auf eine Glasfritte abgelassen und die Toluolphase abgetrennt. Der Filterrückstand wird mit Hexan gewaschen und zunächst bei Raumtemperatur vorgetrocknet. Zur Endtrocknung wird der Feststoff in einen 1 L-Rundkolben gefüllt und im Vakuum bei 110°C getrocknet.

Es werden 338 g (93 % d. Th.) eines granulierten Materials mit einem Staubanteil von 1,5 % erhalten.

### Beispiel 2: Herstellung eines Magnesiumethoxid-Granulates durch Umsetzung von Magnesiumgranalien, Qualität B, mit Ethanol in Toluolsuspension:

In derselben Apparatur wie in Beispiel 1 wird die Magnesiumqualität B mit Ethanol in Toluol umgesetzt. Die Reaktionsbedingungen und -ergebnisse werden in den Tabellen 3 und 4 festgehalten.

### Beispiel 3: Herstellung eines Magnesiumethoxid-Granulates durch Umsetzung von Magnesiumspänen, Qualität C, mit Ethanol in Toluolsuspension

In derselben Apparatur wie in Beispiel 1 wird die Magnesiumqualität C mit Ethanol in Toluol umgesetzt. Die Reaktionsbedingungen und -ergebnisse werden in den Tabellen 3 und 4 festgehalten.

**Tabelle 3: Herstellung von Magnesiumethoxid-Granulat**

| Beispiel | Magnesium | | Toluol | Ethanol | | TEA | |
|---|---|---|---|---|---|---|---|
| | Art | Menge (g) | (g) | (g) | (% d.Th.) | (g) | (mol %) |
| 1 | A | 77,3 | 1400 | 307 | 105 | 2,5 | 0,7 |
| 2 | B | 80,5 | 1550 | 355 | 116 | 1,1 | 0,3 |
| 3 | C | 65,3 | 1630 | 267 | 108 | 2,3* | 1,2* |
| Vgl. 1 | A | 76,0 | 0 | 2050 | 712 | - | - |

| | | | | | | | |
|---|---|---|---|---|---|---|---|
| * Trimethylaluminium | | | | | | | |

**Tabelle 4: Herstellergebnisse**

| Beispiel | Reaktionszeit | Reaktionstemp. | Gasmenge | Produktmenge | Aussehen | Staubanteil |
|---|---|---|---|---|---|---|
| | (h) | (°C) | L | (g) | | (%) |
| 1 | 3,2 | 50 - 90 | 75,8 | 338 | Granulat | 1,5 |
| 2 | 8 | 60 - 105 | 80,1 | 356 | Granulat | <1 |
| 3 | 4 | 90 - 108 | 64,3 | 281 | Granulat | 3 |
| Vgl. 1 | 20 | 50 - 78 | 65,5 | 305 | Pulver mit Klumpen | 72 |

Gespantes Magnesium benötigt nach dem erfindungsgerriäßen Verfahren etwa 3 bis 4 Stunden für die vollständige Umsetzung zu Magnesiumethoxid. Wird kompakteres, granuliertes Magnesiummetall eingesetzt, sind die Reaktionszeiten länger, wie aus Beispiel 2 hervorgeht. Der Staubanteil liegt unter einem %.

### Vergleichsbeispiel 1: Herstellung von Magnesiumethoxid durch Umsetzung von Magnesium in wasserfreien Ethanol:

In der Apparatur aus Beispiel 1 werden 76 g Magnesiumfeinspäne in 2,05 kg wasserfreiem Ethanol (Wassergehalt nach Karl Fischer: 36 ppm) suspendiert und die Manteltemperatur innerhalb von 20 Minuten auf 90°C angehoben, so dass der Reaktorinhalt siedet. Nach insgesamt 70 Minuten haben sich knapp 20 L Gas (ca. 26 % d.Th.) entwickelt. Danach nimmt die Gasbildungsrate stark ab. Innerhalb weiterer 4 Stunden Rückflusskochen werden nur 21 L Gas freigesetzt. Daraufhin werden 0,8 g lod und nach einer weiteren Stunde 1,8 g Eisenchlorid zugegeben. Die Gasbildungsrate nahm daraufhin nur kurzzeitig zu. Nach insgesamt 15- bzw. 20-stündigem Refluxieren haben sich 57,6 bzw. 65,5 L Gas gebildet (ca. 77 bzw. 87 % d.Th.).

Die grau-weiße Suspension wird filtriert und bei 110°C vakuumgetrocknet. Das fertige Produkt besteht überwiegend aus einem feinen Pulver mit sehr hohem Staubanteil. Neben Klumpen enthält es noch etwa 10 % nicht umgesetztes metallisches Magnesium.

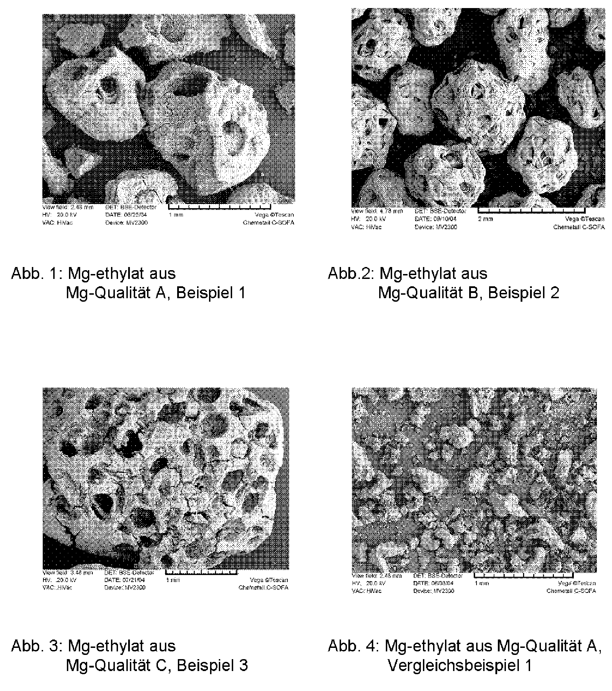

## Patentansprüche

1. Verfahren zur Herstellung eines Magnesiumalkoxidgranulates **dadurch gekennzeichnet, dass** Magnesium mit Magnesiumpartikelgrößen von 0,3 bis 5 mm mit 0,001 bis über 20 mol% mit einer Verbindung oder mehreren Verbindungen der allgemeinen Formel AlR₃₋ₙHalₙ, wobei
- der Rest R als Alkyl- oder Aryl- Rest
- der Rest Hal als Halogen-Rest und
- n als Zahl, wobei 0 ≤ n ≤ 2 ist, aktiviert und einem Überschuss an Alkohol zwischen 0,1 und 60 % in nicht-koordinierenden Lösemitteln umgesetzt wird
mit folgenden Verfahrensschritten:
- Vorlage des Lösemittels und des Magnesiummetalls in einen mit Schutzgas gefüllten Rührreaktor;
- Zugabe einer oder mehrerer Verbindungen der allgemeinen Formel AlR₃₋ₙHalₙ;
- Zugabe des Alkohols.

2. Verfahren nach Anspruch 41, **dadurch gekennzeichnet, dass** das Magnesium in metallischer Form vorgelegt wird.

3. Verfahren nach Anspruch 1 oder 2, **dadurch gekennzeichnet, dass** das Magnesium in granulierter, sphärischer Form vorgelegt wird.

4. Verfahren nach mindestens einem der Ansprüche 1 bis 3, **dadurch gekennzeichnet, dass** die Korngröße des Magnesiums zwischen 1 bis 3 mm beträgt.

5. Verfahren nach mindestens einem der Ansprüche 1 bis 4, **dadurch gekennzeichnet, dass** der Alkohol nach den Verbindungen der allgemeinen Formel AlR₃₋ₙHalₙ zugegeben wird.

6. Verfahren nach mindestens einem der Ansprüche 1 bis 5, **dadurch gekennzeichnet, dass** der Alkohol ein primärer Alkohol ist.

7. Verfahren nach mindestens einem der Ansprüche 1 bis 6, **dadurch gekennzeichnet, dass** der Alkohol ausgewählt ist aus Methanol, Ethanol oder Propanol oder Mischungen aus mindestens zwei dieser Alkohole.

8. Verfahren nach mindestens einem der Ansprüche 1 bis 7, **dadurch gekennzeichnet, dass** der Alkohol Ethanol ist.

9. Verfahren nach mindestens einem der Ansprüche 1 bis 8, **dadurch gekennzeichnet, dass** der Überschuss des Alkohols zwischen ein und 20 %, beträgt.

10. Verfahren nach mindestens einem oder mehreren der Ansprüche 1 bis 9, **dadurch gekennzeichnet, dass** das Lösemittel ausgewählt ist Benzol, Toluol, Ethylbenzol, Cumol, Xylolen oder Mischungen aus mindestens zwei dieser Lösemittel.

11. Verfahren nach mindestens einem der Ansprüche 1 bis 10. **dadurch gekennzeichnet, dass** das Lösemittel Toluol ist.

12. Verfahren nach mindestens einem der Ansprüche 1 bis 11, **dadurch gekennzeichnet, dass** die Zugabe des Aktivierungsmittels bei Temperaturen zwischen 0 und 150°C erfolgt.

13. Verfahren nach mindestens einem der Ansprüche 1 bis 12, **dadurch gekennzeichnet, dass** der Zeitraum, in dem der Alkohol zudosiert wird, zwischen 3 Minuten und 6 Stunden beträgt.

14. Verfahren nach mindestens einem der Ansprüche 1 bis 13, **dadurch gekennzeichnet, dass** die Umsetzung des Magnesiums mit dem Alkohol bei Temperaturen zwischen Raumtemperatur und dem Siedepunkt des Lösemittels erfolgt.

## Claims

1. Process for the preparation of a magnesium alkoxide granulate, **characterized in that** magnesium having magnesium particle sizes of from 0.3 to 5 mm is activated with 0.001 to more than 20 mol% with a compound or several compounds of the general formula AlR₃₋ₙHalₙ, wherein
- the radical R is an alkyl or aryl radical
- the radical Hal is a halogen radical and
- n is a number, wherein 0 ≤ n ≤ 2, and reacted with an excess of alcohol of between 0.1 and 60 % in non-coordinating solvents
with the following process steps:
- initial introduction of the solvent and the magnesium metal into a stirred reactor which has been filled with an inert gas;
- addition of one or more compounds of the general formula AlR₃₋ₙHalₙ;
- addition of the alcohol.

2. Process according to claim 1, **characterized in that** the magnesium is introduced in a metallic form.

3. Process according to claim 1 or 2, **characterized in that** the magnesium is introduced in a granulated, spherical form.

4. Process according to at least one of claims 1 to 3, **characterized in that** the grain size of the magnesium is between 1 to 3 mm.

5. Process according to at least one of claims 1 to 4, **characterized in that** the alcohol is added after the compounds of the general formula AlR₃₋ₙHalₙ.

6. Process according to at least one of claims 1 to 5, **characterized in that** the alcohol is a primary alcohol.

7. Process according to at least one of claims 1 to 6, **characterized in that** the alcohol is chosen from methanol, ethanol or propanol or mixtures of at least two of these alcohols.

8. Process according to at least one of claims 1 to 7, **characterized in that** the alcohol is ethanol.

9. Process according to at least one of claims 1 to 8, **characterized in that** the excess of the alcohol is between one and 20 %.

10. Process according to at least one or more of claims 1 to 9, **characterized in that** the solvent is chosen from benzene, toluene, ethylbenzene, cumene, xylenes or mixtures of at least two of these solvents.

11. Process according to at least one of claims 1 to 10, **characterized in that** the solvent is toluene.

12. Process according to at least one of claims 1 to 11, **characterized in that** the addition of the activating agent is carried out at temperatures of between 0 and 150 °C.

13. Process according to at least one of claims 1 to 12, **characterized in that** the period of time during which the alcohol is metered in is between 3 minutes and 6 hours.

14. Process according to at least one of claims 1 to 13, **characterized in that** the reaction of the magnesium with the alcohol is carried out at temperatures of between room temperature and the boiling point of the solvent.

## Revendications

1. Procédé de préparation d'un granulat d'alcoolate de magnésium, **caractérisé en ce que** l'on active du magnésium, en particules de 0,3 à 5 mm de taille, avec de 0,001 à plus de 20 % en moles d'un composé ou de plusieurs composés de formule générale AlR₃₋ₙHalₙ dans laquelle
- le symbole R représente un groupe alkyle ou aryle,
- le symbole Hal représente un atome d'halogène,
- et l'indice n est un nombre tel que 0 ≤ n ≤ 2,
et on le fait réagir avec un alcool en excès de 0,1 à 60 %, dans un solvant non-coordinant, en procédant aux opérations suivantes :
- on met le solvant et le métal magnésium dans un réacteur agité rempli d'un gaz de protection,
- on ajoute un ou plusieurs composé(s) de formule générale AlR₃₋ₙHalₙ,
- et l'on ajoute l'alcool.

2. Procédé conforme à la revendication 1, **caractérisé en ce que** le magnésium se présente sous forme métallique.

3. Procédé conforme à la revendication 1 ou 2, **caractérisé en ce que** le magnésium se présente sous forme de granules sphériques.

4. Procédé conforme à l'une au moins des revendications 1 à 3, **caractérisé en ce que** la taille des particules de magnésium vaut de 1 à 3 mm.

5. Procédé conforme à l'une au moins des revendications 1 à 4, **caractérisé en ce qu'**on ajoute l'alcool après les composés de formule générale AlR₃₋ₙHalₙ.

6. Procédé conforme à l'une au moins des revendications 1 à 5, **caractérisé en ce que** l'alcool est un alcool primaire.

7. Procédé conforme à l'une au moins des revendications 1 à 6, **caractérisé en ce que** l'alcool est choisi parmi les méthanol, éthanol et propanol et les mélanges d'au moins deux de ces alcools.

8. Procédé conforme à l'une au moins des revendications 1 à 7, **caractérisé en ce que** l'alcool est de l'éthanol.

9. Procédé conforme à l'une au moins des revendications 1 à 8, **caractérisé en ce que** l'excès d'alcool vaut de 1 à 20 %.

10. Procédé conforme à l'une au moins des revendications 1 à 9, **caractérisé en ce que** le solvant est choisi parmi les benzène, toluène, éthyl-benzène, cumène et xylènes et les mélanges d'au moins deux de ces solvants.

11. Procédé conforme à l'une au moins des revendications 1 à 10, **caractérisé en ce que** le solvant est du toluène.

12. Procédé conforme à l'une au moins des revendications 1 à 11, **caractérisé en ce qu'**on effectue l'addition de l'agent d'activation à une température comprise entre 0 °C et 150 °C

13. Procédé conforme à l'une au moins des revendications 1 à 12, **caractérisé en ce qu'**on ajoute l'alcool en un laps de temps compris entre 3 minutes et 6 heures.

14. Procédé conforme à l'une au moins des revendications 1 à 13, **caractérisé en ce que** la réaction du magnésium avec l'alcool se déroule à une température comprise entre la température ambiante et le point d'ébullition du solvant.
